# EUROPEAN PATENT APPLICATION

(11) **EP 1 596 444 A1**
(43) Date of publication of application: **16.11.2005**
(21) Application number: 04711727.0
(22) Date of filing: 17.02.2004
(51) Int. Cl.: H01L 51/00, H01L 29/43, H01L 29/47, H01L 29/872, H01L 21/28

(54) **RECTIFIER ELEMENT**

(30) Priority: 17.02.2003 JP 2003038720; 17.04.2003 JP 2003112434
(71) Applicant: FUJI ELECTRIC HOLDINGS CO., LTD., Kawasaki-shi, Kanagawa 210-0856 (JP)
(72) Inventor: KAWAKAMI, Haruo, C/o Fuji Elec. Adv. Tech. Co. Ltd, Yokosuka-shi, Kanagawa 240 0194 (JP); KATO, Hisato, C/o Fuji Electric Adv. Tech. Co. Ltd, Yokosuka-shi, Kanagawa 240-0194 (JP); YAMASHIRO, Keisuke, C/o Fuji El. Adv. Tech. CoLtd, Yokosuka-shi, Kanagawa 240-0194 (JP); KURODA, Masami, C/o Fuji Elec. Adv. Tech. Co. Ltd., Yokosuka-shi, Kanagawa 240-0194 (JP); SEKINE, Nobuyuki, C/o Fuji Elec. Adv. Tech. Co Ltd, Yokosuka-shi, Kanagawa 240-0194 (JP)
(74) Representative: Appelt, Christian W.
(86) International application number: PCT/JP2004/001718
(87) International publication number: WO 2004/073080

(57) **Abstract**

The present invention provides a rectifying element making it possible to obtain a stable rectification characteristic with a simple structure.

A first electrode layer, an organic semiconductor material layer, and a second electrode layer are successively formed as thin films on a substrate. The organic semiconductor material constituting the organic semiconductor material layer is a quinomethane-based compound or a monoquinomethane-based compound. The first electrode and second electrode are preferably formed of the same material and even more preferably of aluminum.

## Description

### TECHNICAL FIELD

The present invention relates to a rectifying element using an organic semiconductor that can be advantageously employed, for example, in organic light-emitting displays or organic solar cells, and more particularly to a two-terminal element demonstrating a nonlinear voltage-current characteristic.

### BACKGROUND ART

In recent years, characteristics of organic electronic materials have improved remarkably, and organic light-emitting displays, organic solar cells, organic thin-film transistors, and organic fuel cells employing organic semiconductors have been extensively researched. Because diodes that are basic components thereof occupy an important place as electronic devices with a rectifying action, they have been studied especially extensively.

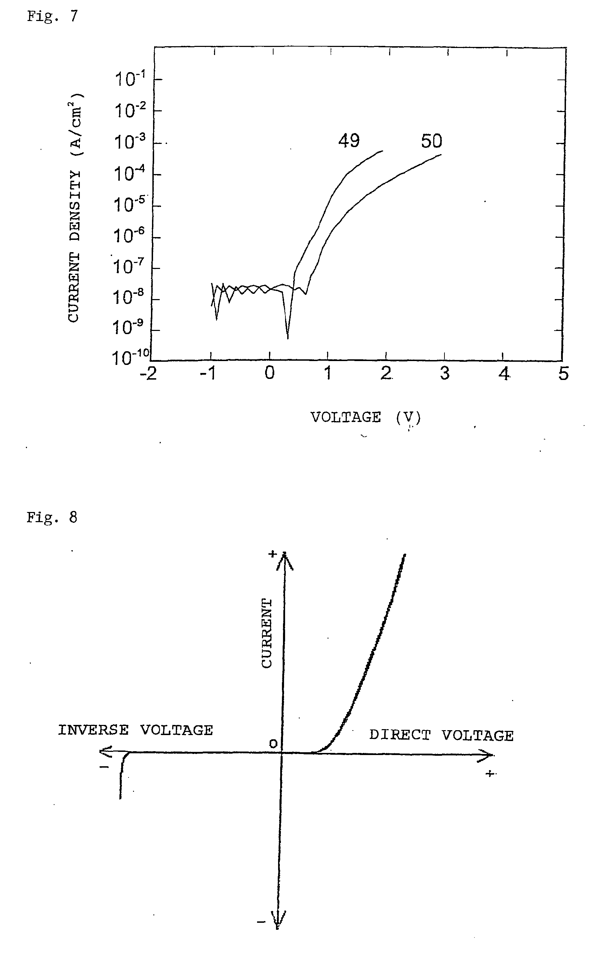

FIG. 8 shows a rectification characteristic of the voltage-current characteristic in such a diode. As shown in FIG. 8, when a positive voltage is applied to electrodes, an ohmic connection is formed such that an electric current starts flowing as a certain threshold voltage is exceeded. Conversely, when a negative voltage is applied, practically no current flows and the material behaves as an insulator.

There are three types of mechanisms for obtaining such rectification characteristic: based on a pn junction, based on a Schottky junction of a metal and a semiconductor, and a method using the difference in work function between the metals of two types sandwiching an insulator. Generally, when an organic semiconductor with low mobility is used, there are two main mechanisms: based on a pn junction and based on a Schottky junction of a metal and a semiconductor.

Organic light-emitting displays employing organic light-emitting diodes that have been studied recently on a large scale represent an example of obtaining a rectification characteristic by using a pn junction. For example, JP-A-2002-289353, 2002-289355, and 2002-289878 disclose organic semiconductor diodes comprising a hole transport layer comprising an organic compound having a hole transport ability on the anode side and an electron transport layer comprising an organic compound having an electron transporting ability on the cathode side and having a non-linear current-voltage characteristic when a voltage is applied between the adjacent hole transport layer and electron transport layer. It is disclosed that the hole transport layer uses a porphirin derivative and the electron transport layer uses a perylene derivative.

Further, as an example of using a Schottky junction, T. Aernouts et al. disclosed a Schottky diode of a three-layer structure in which a PPV (polyphenylenevinylene) is sandwiched between ITO and aluminum and reported a rectification characteristic originating on the interface of the PPV oligomer and aluminum electrode (T. Aernouts et al. Synthetic Metals, Vol. 122, pp. 153, 2001).

However, the following problems are associated with the diodes using such organic semiconductors.

Thus, the problem arising when a pn junction is used, as for the organic semiconductor diode in JP-A-2002-289353, 2002-289355, and 2002-289878, is that at least four layers: a first electrode, a p-type semiconductor, a n-type semiconductor, and a second electrode are necessary and the process is complex.

Further, the problem arising when a Schottky junction is used is that though the structure is simple because only one organic layer can be used, the organic semiconductor material used, such as PPV, is extremely sensitive to oxygen or moisture and is very difficult to handle.

Furthermore, the problem arising when two electrodes are from dissimilar metals is that a contact difference in electric potential is generated when they are laminated and the resultant physical properties are inappropriate for sensors and solar cells using a photoelectromotive force.

The present invention was created to resolve the above-described problems of the conventional technology and it is an object thereof to provide a rectifying element making it possible to obtain a stable rectification characteristic with a simple structure.

### DISCLOSURE OF THE INVENTION

One rectifying element in accordance with the present invention is a rectifying element in which an organic semiconductor material is disposed between at least two electrodes, wherein
a first electrode layer, an organic semiconductor material layer, and a second electrode layer are successively formed as thin films on a substrate and the organic semiconductor material constituting the organic semiconductor material layer is a quinomethane-based compound represented by the general formula (I) below. 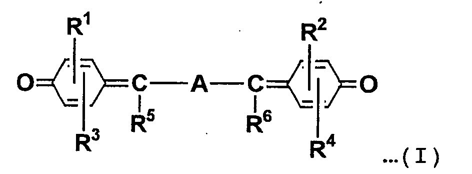 (in Formula (I), each of R¹-R⁴ represents a group selected from a hydrogen atom, an optionally substituted C₁₋₆ alkyl group, and an optionally substituted aryl group, where R¹-R⁴ may be same or different. Each of R⁵ and R⁶ represents an optionally substituted aryl group or an optionally substituted hetero ring, where R⁵, R⁶ may be same or different. A represents a group selected from groups (1)-(10) shown below). 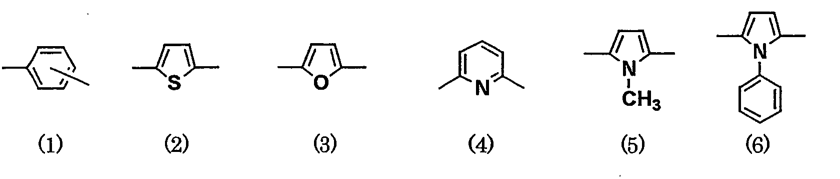 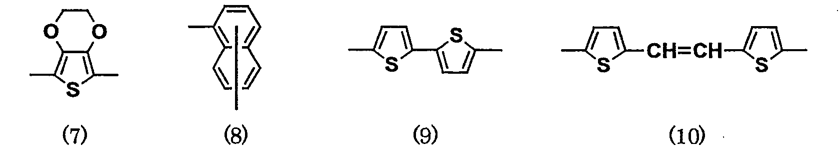
with the above-described rectifying element, the element structure is simple. Furthermore, because the quinomethane compound represented by the general formula (I) above has strong affinity to electrode materials, in particular when an electrode is vapor deposited on a thin film of the quinomethane-based compound, a mixed layer of the electrode and quinomethane-based compound is formed. This is supposedly why the below described electrically asymmetric rectification characteristic can be obtained.

Another rectifying element in accordance with the present invention is a rectifying element in which an organic semiconductor material is disposed between at least two electrodes, wherein a first electrode layer, an organic semiconductor material layer, and a second electrode layer are successively formed as thin films on a substrate and the organic semiconductor material constituting the organic semiconductor material layer is a monoquinomethane-based compound represented by the general formula (II) below. 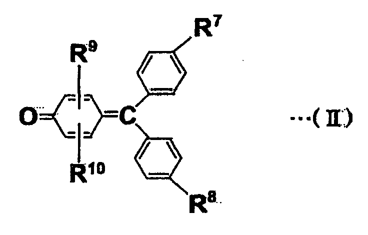 (in Formula (II), each of R⁷ and R⁸ represents a hydrogen atom, a halogen atom, a C₁₋₆ alkyl group, or an alkoxyl group, where R⁷ and R⁸ may be same or different; each of R⁹ and R¹⁰ represents a hydrogen atom, an optionally substituted C₁₋₆ alkyl group, or an optionally substituted aryl group, where R⁹ and R¹⁰ may be same or different).

With the above-described rectifying element, the element structure is simple. Furthermore, because the monoquinomethane compound represented by the general formula (II) above has strong affinity to electrode materials, in particular when an electrode are vapor deposited on a thin film of the monoquinomethane-based compound, a mixed layer of the electrode and monoquinomethane-based compound is formed. This is supposedly why the below described electrically asymmetric rectification characteristic can be obtained.

Yet another rectifying element in accordance with the present invention is a rectifying element in which an organic semiconductor material is disposed between at least two electrodes, wherein
a first electrode layer, an organic semiconductor material layer, and a second electrode layer are successively formed as thin films on a substrate and the organic semiconductor material constituting said organic semiconductor material layer is a monoquinomethane-based compound represented by the general formula (III) below. 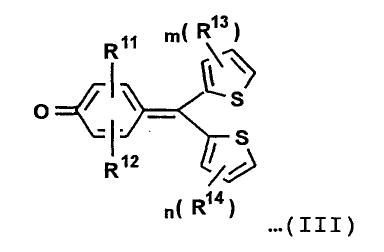 (in Formula (III), each of R¹¹-R¹⁴ represents a group selected from a hydrogen atom, a halogen atom, an optionally substituted C₁₋₆ alkyl group, and an optionally substituted aryl group, where R¹¹-R¹⁴ may be same or different; m and n are integers, each from 0 to 3).

With the above-described rectifying element, the element structure is simple. Furthermore, because the monoquinomethane compound represented by the general formula (III) above has strong affinity to electrode materials, in particular when an electrode are vapor deposited on a thin film of the monoquinomethane-based compound, a mixed layer of the electrode and monoquinomethane-based compound is formed. This is supposedly why the below described electrically asymmetric rectification characteristic can be obtained.

In the rectifying elements in accordance with the present invention, the first electrode and the second electrode are preferably from the same material.

In this case, because the identical materials are used for the first electrode and second electrode, the problem associated with the difference in contact electric potential during lamination is not encountered. This result is advantageous from the standpoint of sensors and solar cells using a photoelectromotive force. As for electric characteristics obtained when the first electrode and second electrode are of the same material, it has been conventionally thought that a characteristic symmetrical with respect to the polarity of the applied voltage is demonstrated, but it was found that if an element is formed by disposing an organic semiconductor material between at least two electrodes by a vacuum vapor deposition method, the energy level or mode will differ between the interface state of the first electrode and organic semiconductor material and the interface state of the organic semiconductor material and second electrode. This difference is caused by mutual diffusion or chemical reactions of the materials, in particular, induced by the effect of heat or impact energy of the second electrode material landing on the thin film of organic material when the second electrode is formed.

In the rectifying element in accordance with the present invention, the first electrode and the second electrode are preferably from aluminum

In this case, because the quinomethane-based compound of general formula (I) or monoquinomethane-based compounds of general formulas (II), (III) that are the aforementioned organic semiconductor materials have an especially strong affinity to aluminum, when aluminum is vapor deposited on a thin film of the organic semiconductor material, a mixed layer of the aluminum and organic semiconductor material is easily formed. As a result, the below described electrically asymmetrical rectification characteristic is obtained.

### BRIEF DESCRIPTION OF THE DRAWINGS

FIG. 1 is a drawing illustrating an embodiment of the rectifying element in accordance with the present invention; (a) is a schematic plan view, (b) is a schematic cross-sectional view.
FIG. 2 is a diagram illustrating the distribution of aluminum in an embodiment of the rectifying element of the present invention;
FIG. 3 is a diagram illustrating the results obtained in measuring a vacuum level shift in the mixed layer of quinomethane and aluminum used in the rectifying element of the present invention;
FIG. 4 is an explanatory drawing illustrating the energy state of each layer in an embodiment of the rectifying element of the present invention;
FIG. 5 is a diagram showing the current-voltage characteristic of the rectifying elements in working Examples 1 and 2;
FIG. 6 is a diagram showing the current-voltage characteristic of the rectifying elements in Working Examples 3 to 7;
FIG. 7 is a diagram showing the current-voltage characteristic of the rectifying elements in Working Examples 8 and 9;
FIG. 8 is a diagram showing the voltage-current characteristic of the conventional rectifying element.

### BEST MODE FOR CARRYING OUT THE INVENTION

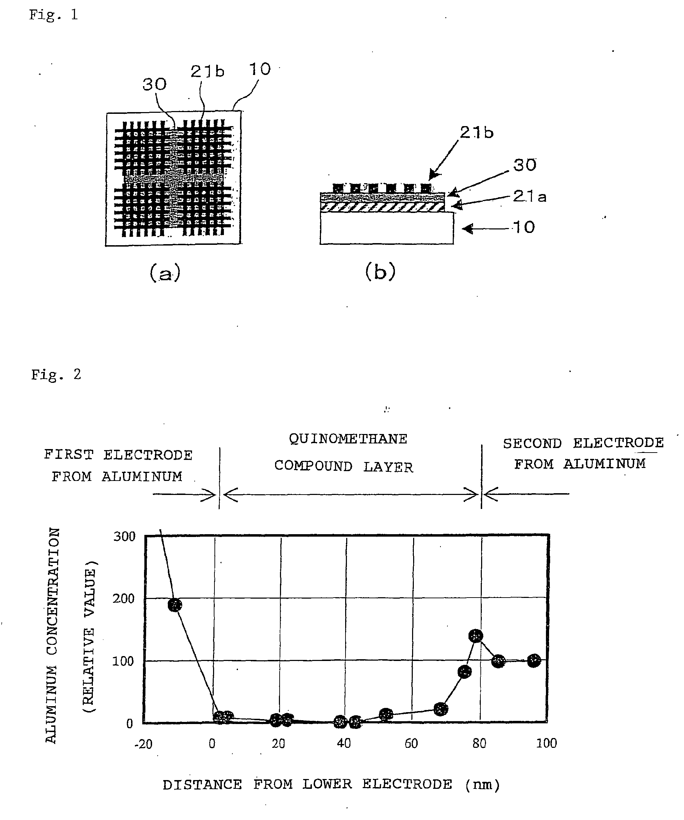

The present invention will be described below in greater detail with reference to the drawings. FIG. 1 is a schematic structural drawing illustrating an embodiment of the rectifying element of the present invention.

As shown in FIG. 1(b), in the rectifying element, a first electrode layer 21a, an organic semiconductor material layer 30, and a second electrode layer 21b are successively laminated as thin films on a substrate 10. The second electrode layer 21b is formed to have a grid-like pattern, as shown in FIG. 1(a).

No specific limitation is placed on the material for the substrate 10, but a conventional well-known glass substrate is preferably used therefor.

The conventional well-known methods such as vacuum vapor deposition method or sputtering method can be used for forming thin films of the first electrode layer 21a and second electrode layer 21b. Those film forming methods can be implemented not only in a usual manner under vacuum, but also by introducing a reactive gas such as oxygen, moisture, or nitrogen into the vacuum atmosphere, or physical properties of the film-forming material can be controlled by applying an electric field or inducing an electric discharge in the vacuum atmosphere. The usual direct current method and RF method can be used as sputtering methods, but those methods are not limiting.

Further, no specific limitation is placed on the method for forming the organic semiconductor material layer 30, and methods for manufacturing organic thin film such as a spin coating method, an electrolytic polymerization method, a chemical vapor deposition method (CVD method), and a monomolecular film deposition method (LB method) can be used. A vacuum vapor deposition method that can employ a film forming method identical to that of the above-described electrode layers is preferably used.

The substrate temperature during vacuum vapor deposition or sputtering is appropriately selected according to the electrode material and organic semiconductor material used. When the first and second electrode layers 21a, 21b are formed, the temperature is preferably 0-150°C, and when the organic semiconductor material layer 30 is formed, the temperature is preferably 0-100°C.

As for the thickness of each layer, it is preferred that the thickness of the first electrode layer 21a and second electrode layer 21b be 50-200 nm and the thickness of the organic semiconductor material layer 30 be 20-150 nm.

The second electrode layer 21b may be laminated over the entire surface of the organic semiconductor material layer 30, or may be patterned as a grid, as shown in FIG. 1(b). For example, when a grid-like pattern is formed, the line width is preferably 0.1-1000 µm and the grid pitch is preferably 0.2-3000 µm.

The materials of the first electrode layer 21a and second electrode layer 21b may be selected so as to facilitate the interaction with the organic material and so that the absolute value of the work function of electrode layers be advantageous for charge transport into the organic semiconductor material. When the organic semiconductor material has an electron transporting ability, a metal material, or an inorganic material and a semiconductor material having a low work function can be appropriately selected. When the organic semiconductor material has a hole transporting ability, a metal material, or an inorganic material and a semiconductor material having a high work function can be appropriately selected and no specific limitation is placed thereon.

Here the term "work function" stands for a minimum energy necessary to remove an electron from the surface of a certain material. It is a value inherent to the electrode material. The work function can be measured by a photoelectron emission spectrum in atmosphere.

Examples of electrode materials with a low work function include aluminum, lithium, magnesium, and calcium. Examples of materials with a high work function include gold, chromium, copper, nickel, platinum, tungsten, and ITO.

Among them, aluminum is preferably used because it easily interacts with the below-described quinomethane-based compound of general formula (I) and monoquinomethane-based compounds of general formulas (II) and (III). It is further preferred that the first electrode layer 21a and second electrode layer 21b be from the same materials.

The organic semiconductor material layer 30 is formed as a thin film on the first electrode layer 21a. A compound having a functional group for transporting the electric charge and comprising an electron-donating functional group and an electron-receiving functional group in a molecule is used as the organic semiconductor material used in the organic semiconductor material layer 30.

Examples of electron-donating functional groups include -SCH₃, -OCH₃, -NH₂, -NHCH₃, and -N(CH₃)₂, and examples of electron-accepting functional groups include -CN, NO₂, -CHO, -COCH₃, -COOC₂H₅, -COOH, -Br, -Cl, -I, -OH, -F, and =O.

In accordance with the present invention, a quinomethane-based compound represented by the general formula (I) below is used as a compound having the above-described electron-donating functional group and the above-described electron-accepting functional group in a molecule. 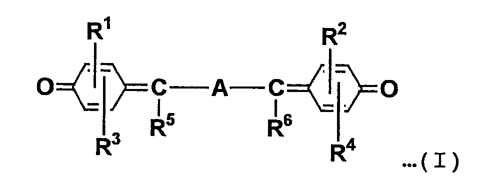 (in Formula (I), each of R¹-R⁴ represents a group selected from a hydrogen atom, an optionally substituted C₁₋₆ alkyl group, and an optionally substituted aryl group, where R¹-R⁴ may be same or different. Each of R⁵ and R⁶ represents an optionally substituted aryl group or an optionally substituted hetero ring, where R⁵, R⁶ may be same or different. A represents a group selected from groups (1)-(10) shown below)

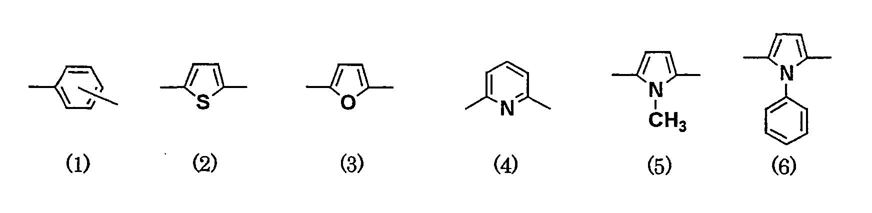

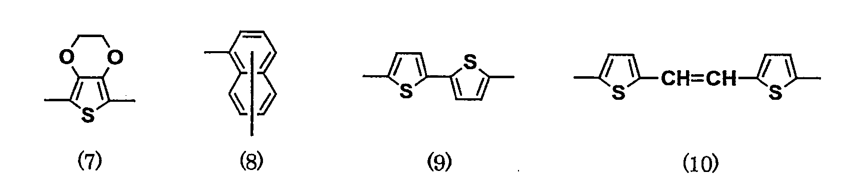

The quinomethane-based compound represented by the general formula (I) is a material comprising an electron-accepting quinone group and having an electron transporting ability.

This quinomethane-based compound (I) can be synthesized according to the reaction formula shown below. The compound shown below is an example with the above-mentioned A being (2) or (3) (X is represents oxygen or sulfur).

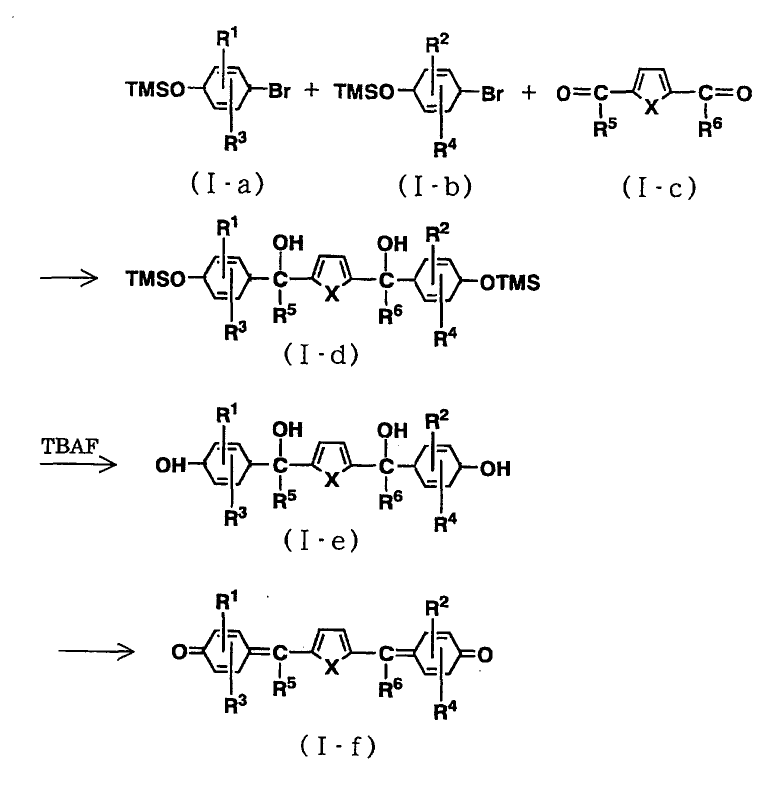

Thus, a compound (I-e) can be synthesized by reacting a compound (I-a) and a compound (I-b) with compound (I-c) in the presence of an appropriate organometallic catalyst, for example, n-butyllithium to obtain a compound (I-d) and removing TMS (trimethylsilyl group) that is a protective group. Then, a quinomethane-based compound (I-f) can be obtained by further subjecting the compound (I-e) to dehydration condensation in the presence of a catalyst, for example, p-toluenesulfonic acid. TBAF in the reaction formula above represents tetrabutylammonium fluoride. This synthesis method is described in detail, for example, in JP-A-2003-228185, 2003-238561, and 2003-105039.

Specific examples of the aforementioned quinomethane-based compound are represented, for example, by the following structural formulas (I-1)-(I-32). 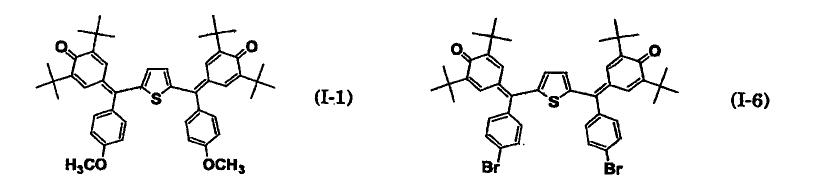 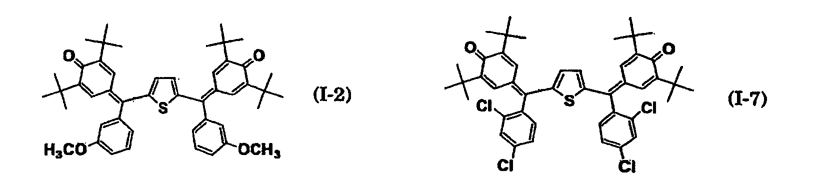 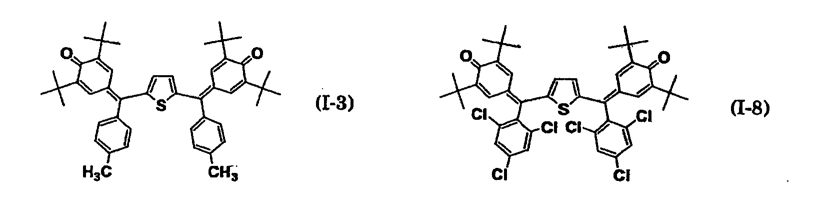 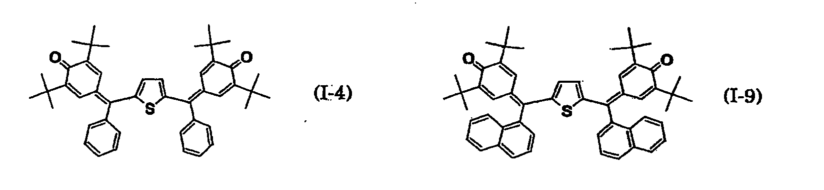 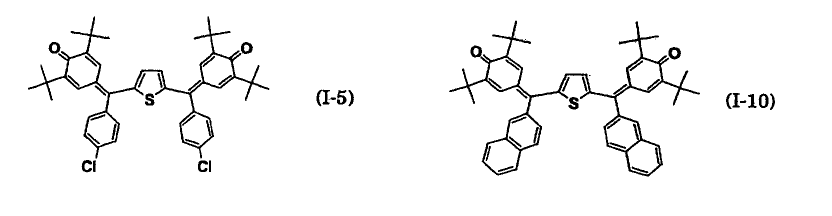 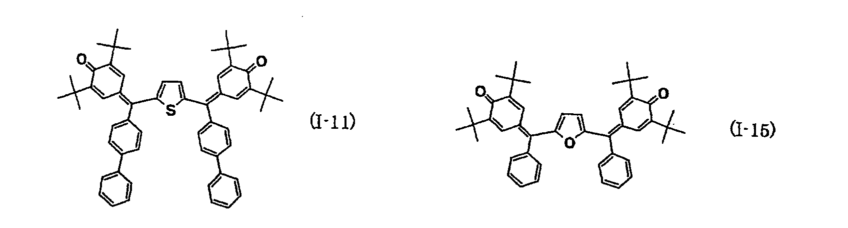 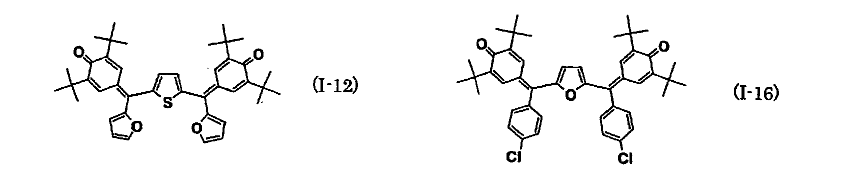 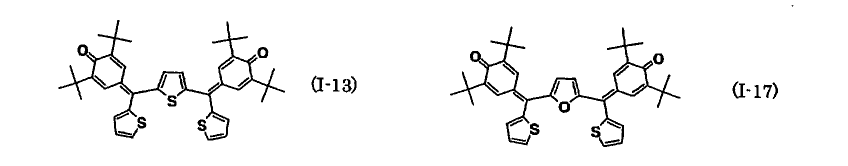 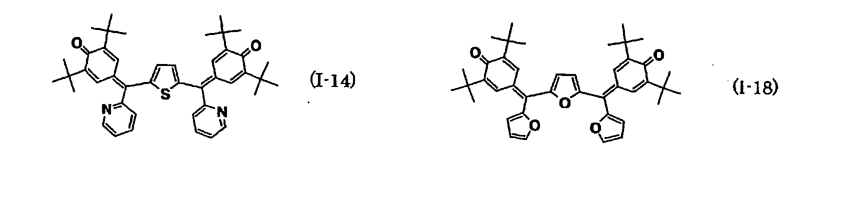 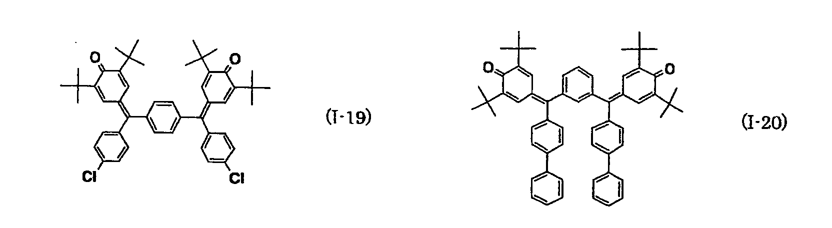 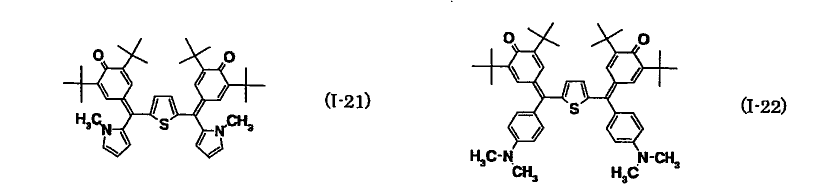 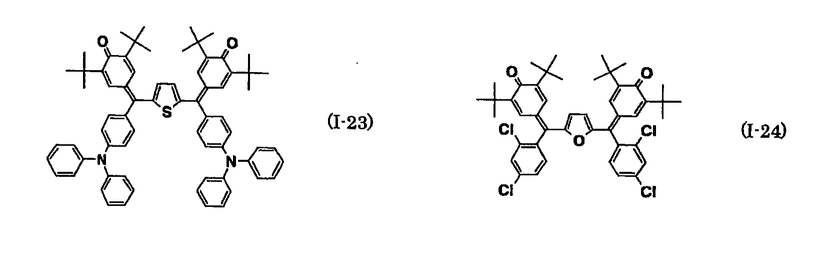 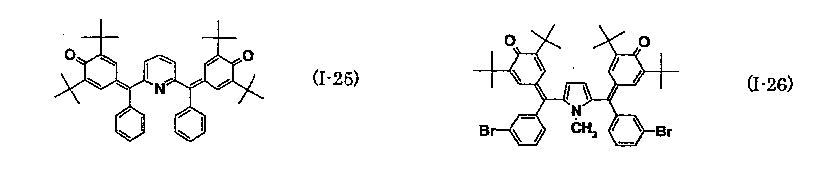 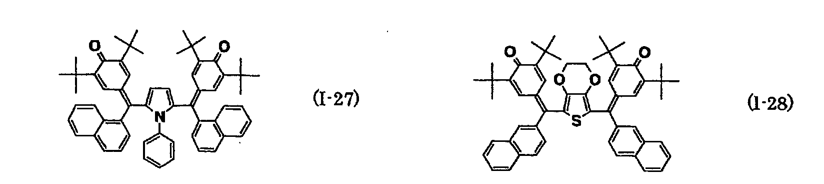 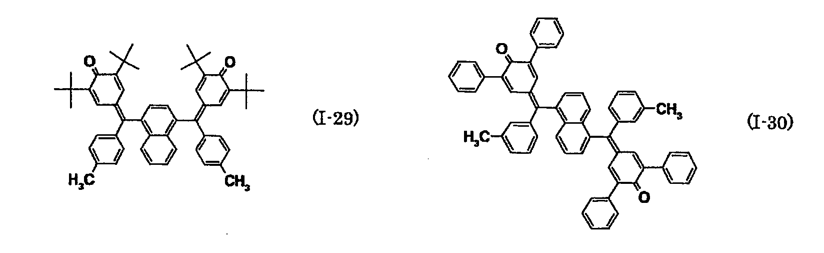 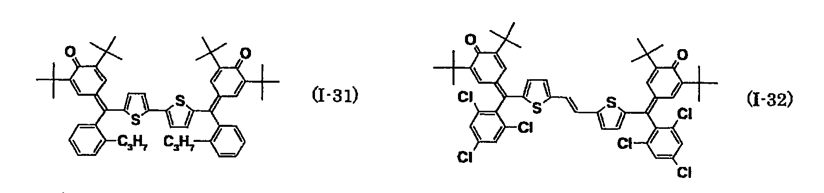 (in the compounds I-1 to I-32 above, symbol "+" of the substitution group represents a t-butyl group).

Further, a monoquinomethane-based compound represented by the following structural formula (II) may be used as the organic semiconductor material. 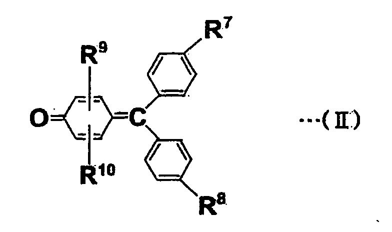 (in Formula (II), each of R⁷ and R⁸ represents a hydrogen atom, a halogen atom, a C₁₋₆ alkyl group, or an alkoxyl group, where R⁷ and R⁸ may be same or different; each of R⁹ and R¹⁰ represents a hydrogen atom, an optionally substituted C₁₋₆ alkyl group, or an optionally substituted aryl group, where R⁹ and R¹⁰ may be same or different).

This monoquinomethane-based compound can be obtained, for example, by the method described in JP-A-8-278643.

The monoquinomethane-based compound represented by the general formula (II) is a material comprising an electron-accepting quinone group and having an electron transporting ability.

Specific examples of the aforementioned monoquinomethane-based compound are represented, for example, by the following structural formulas (II-1) and (II-2). 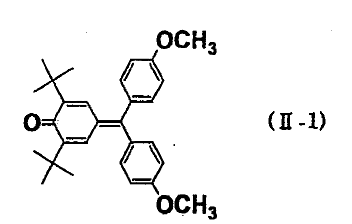 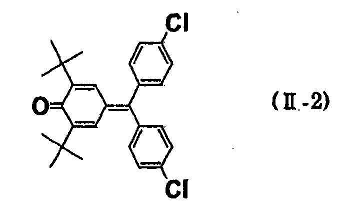 (in the compounds II-1 and II-2 above, symbol "+" of the substitution group represents a t-butyl group).

Further, the monoquinomethane-based compound represented by the general formula (III) below may be also used as the organic semiconductor material. 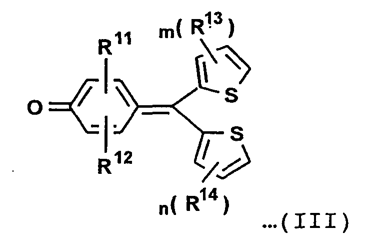 (in Formula (III), each of R¹¹-R¹⁴ represents a group selected from a hydrogen atom, a halogen atom, an optionally substituted C₁₋₆ alkyl group, and an optionally substituted aryl group, where R¹¹-R¹⁴ may be same or different; m and n are integers, each from 0 to 3).

The monoquinomethane-based compound represented by the general formula (III) is a material comprising an electron-accepting quinone group and having an electron transporting ability.

Specific examples of the aforementioned monoquinomethane-based compound are represented, for example, by the following structural formulas (III-1) to (III-12).

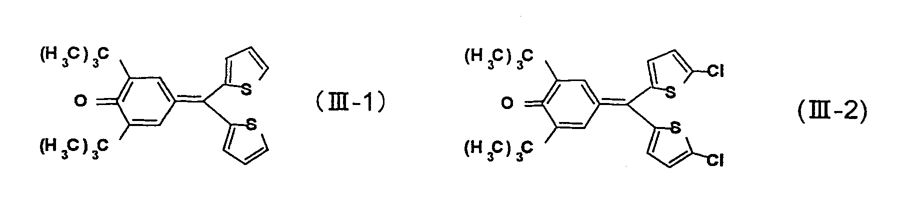

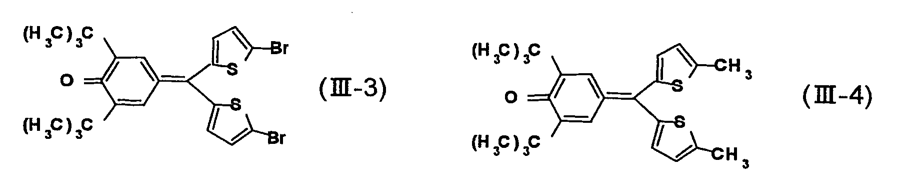

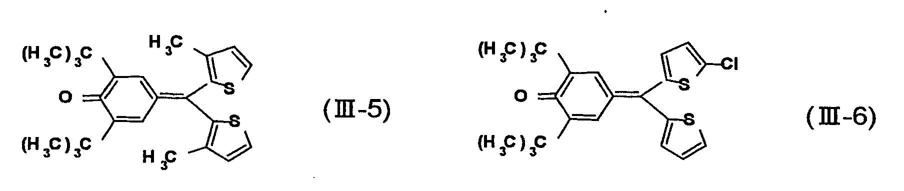

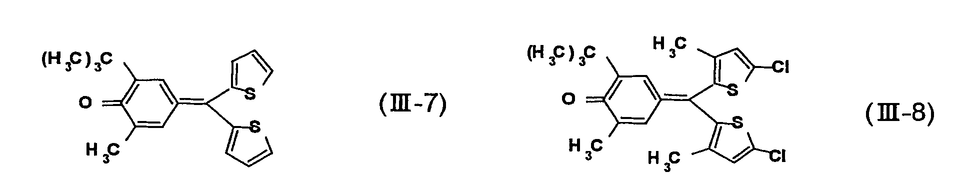

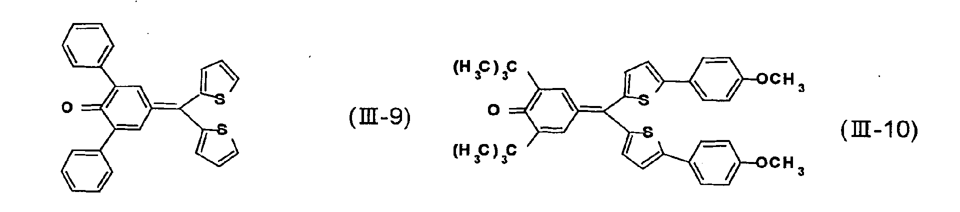

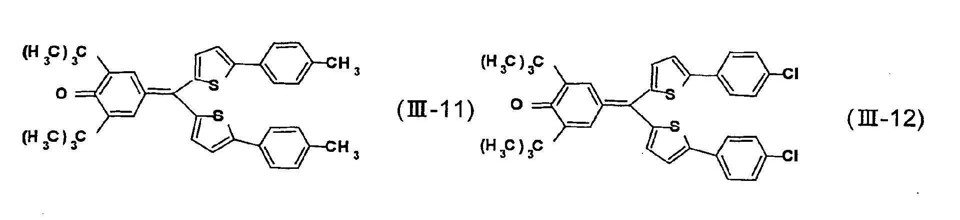

The monoquinomethane-based compound of the general formula (III) shown hereinabove can be synthesized, for example, according the reaction formula shown hereinbelow. The example shown below relates to synthesizing the compound represented by the structural formula (III-1) shown above, but compounds of other structures can be synthesized by the same method.

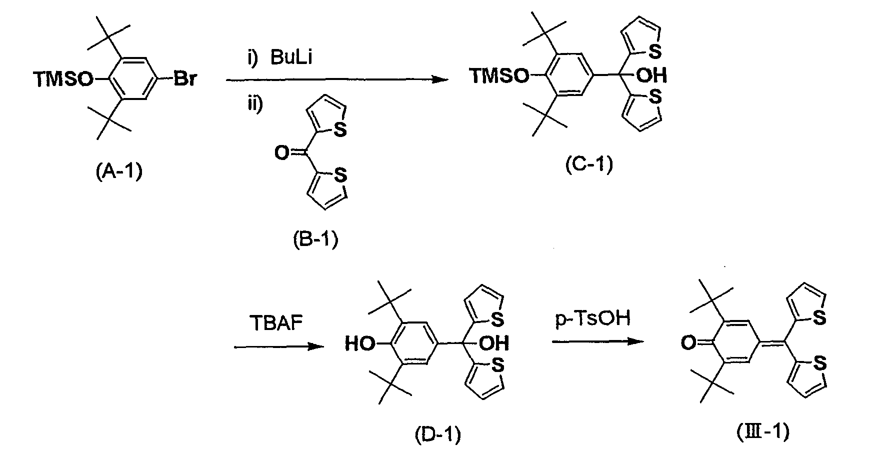

Thus, for example, 29 mmol (18 mL) of 1.6 M n-butyllithium hexane solution (BuLi) is dropwise added under a nitrogen atmosphere at -78°C to a THF solution of 24 mmol (8.6 g) of 4-bromo-2,6-di-t-butyl-1-[trimethylsilyl]benzene (structural formula (A-1)). Then, a compound of structural formula (C-1) is obtained by adding 20 mmol (3.9 g) of dithienyl ketone (structural formula (B-1)) and stirring at room temperature.

An aqueous solution of ammonium chloride and then 24 mmol (24 mL) of 1.0 M THF solution of tetrabutylammonium fluoride (TBAF) are thereafter dropwise added to obtain a compound of structural formula (D-1). Then, a compound of structural formula (III-1) can be obtained by adding a small quantity of p-toluenesulfonic acid monohydrate (p-TsOH), refluxing under heating, distilling the solvent off, and recrystallizing the solids in a mixed solvent of chloroform and hexane.

Here, 4-bromo-2,6-di-tert-butyl-1-[trimethylsilyl]benzene (structural formula (A-1)) can be synthesized, for example, by a well-known method described in JP-A-2003-238561. Furthermore, di-2-thienyl ketone (structural formula (B-1)) is readily available from Sigma-Aldrich Japan Co.

Further, the yield of the compound obtained by the above-described synthesis method was 3.3 g (yield 43.2%), MS m/z 382 (M+) result was obtained by mass spectrometry, and the structure represented by the structural formula (III-1) shown above was confirmed.

The rectifying element in accordance with the present invention that has the above-described configuration has a simple structure and no difference in contact electric potential between the electrodes. This is supposedly due to the following reasons.

Because the quinomethane compound represented by the general formula (I) and the monoquinomethane compounds represented by the general formulas (II) and (III), which are the above-described organic semiconductor materials, have strong affinity to electrode materials, especially to aluminum, for example, when aluminum is vapor deposited on a thin film of the organic semiconductor material, a mixed layer of the aluminum and organic semiconductor material is formed.

FIG. 2 shows the results obtained in studying the distribution of aluminum in the thickness direction of a thin film in the case where thin films of aluminum (first electrode), above-described quinomethane-based compound of structural formula (I-1), and aluminum (second electrode) were vacuum vapor deposited in the order of description as thin films. It is clear that a region where the two components are present was formed on the interface of the quinomethane-based compound and second electrode. This is supposedly why an electrically asymmetrical rectification characteristic is obtained.

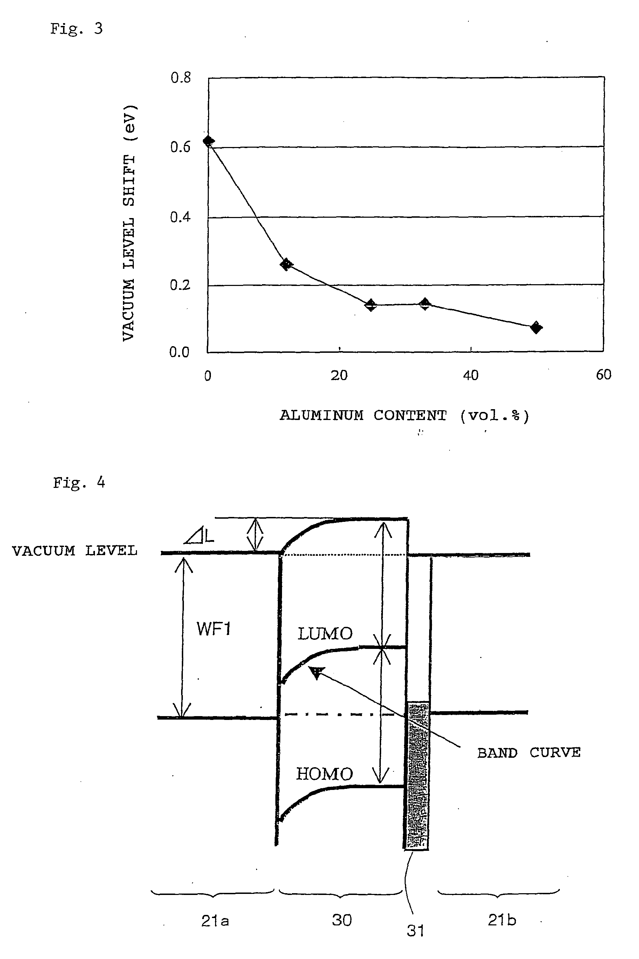

FIG. 3 illustrates the results obtained in studying the relationship between the composition of a mixed layer formed by co-deposition of the quinomethane-based compound of structural formula (I-1) and aluminum and the vacuum level shift quantity on the aluminum film. The vacuum level shift quantity is a relative shift quantity of the energy level caused in particular by a charge shift on the interface of an organic material and a metal material. The vacuum level shift quantity assumes a large value for the quinomethane compound alone and decreases as aluminum is admixed.

FIG. 4 is an energy level diagram of the element in accordance with the present invention that is assumed based on the vacuum level shift quantity. When the first electrode layer 21a is grounded and a positive voltage is applied to the second electrode layer 21b, electrons flow along the gentle curve of the band of conduction levels of the quinomethane compound (organic semiconductor material layer 30), but when a negative voltage is applied to the second electrode layer 21b, a steep wall is raised from a low level of the mixed layer 31 of aluminum and quinomethane-based compound to a high level of the organic semiconductor material level 30. As a result, the electric current is inhibited. This is supposedly why a high rectification ability is realized.

Here, WF₁ stands for a work function of the first electrode layer, LUMO stands for a lowest unoccupied orbital level of the organic semiconductor material layer, and ΔL stands for a vacuum level decrease quantity from the first electrode layer to the organic semiconductor material layer. The highest occupied orbital level (HOMO) of the organic semiconductor material layer can be measured, for example, by a photoelectron emission spectrum in atmosphere. The lowest unoccupied orbital level (LUMO) can be calculated from the optical energy gap obtained from the highest occupied orbital level obtained as described above and an absorption spectrum. The optical energy gap thus obtained is, strictly speaking, somewhat different from the actual energy gap, but this method is widely used as a simple measurement method.

Further, ΔL, which is the vacuum level shift quantity from the first electrode layer to the organic semiconductor material layer, is due to an electric double layer appearing on the interface and strongly affects a charge injection phenomenon. This vacuum level shift quantity can be measured, for example, by the conventional well-known Kelvin method. This measurement method has been described in the publication of Ito et al. (Ito et al. Hyomen, Vol. 38, p. 517 (2000)) . Generally an electric double layer is formed on the interface of thin metal films and thin films of organic semiconductors and this effect is known to be capable of shifting the vacuum level of the metal films and thin films of organic semiconductors (for example, Hyomen, vol. 34, No. 10, p. 621-629, 1996).

As for the band curve, it is generally known that when a semiconductor thin film and an electrode material are brought into contact with each other, the energy level in the semiconductor thin film varies depending on the spatial charge so that the energy balance of the two is maintained (for example, "Handbook on Applied Physics", p. 453, edited by Applied Physics Association, published by Maruzen (1990)).

As described hereinabove, with the present invention, a rectifying element of a simple structure and no difference in contact electric potential between the electrodes can be obtained. Therefore, this rectifying element can be advantageously used in organic light-emitting displays or organic solar cells.

The rectifying element in accordance with the present invention will be described below in greater detail by using working examples thereof.

### Working Example 1

The rectifying element with a configuration shown in FIG. 1 was produced by the following procedure.

The rectifying element of Working Example 1 was formed by using a glass substrate as the substrate 10 and successively and continuously forming thin films by vacuum vapor depositing aluminum as the first electrode layer 21a, a quinomethane-based compound as the organic semiconductor material layer 30, and aluminum as the second electrode layer 21b. The compound with the below-described structural formula (I-1) was used as the quinomethane-based compound.

The second electrode layer 21b was formed to have a grid-like shape with a line width of 0.5 mm and a grid pitch of 2 mm, as shown in FIG. 1(a).

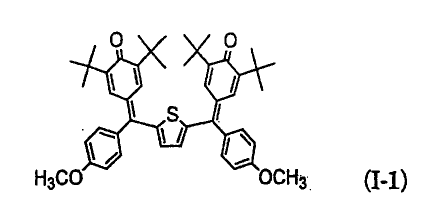

Further, the first electrode layer 21a, organic semiconductor material layer 30, and second electrode layer 21b were formed to have a thickness of 100 nm, 80 nm, and 100 nm, respectively. The vapor deposition apparatus had a diffusion pump gas discharge and the degree of vacuum was 3 x 10⁻⁶ Torr. Vapor deposition of aluminum was conducted by a resistance heating method at a film formation rate of 3 Å/sec, and vapor deposition of quinomethane-based compound was conducted by a resistance heating method at a film formation rate of 2 Å/sec. Vapor deposition of each layer was conducted continuously in the same vapor deposition apparatus under conditions preventing the contact of samples with the air during vapor deposition.

### Working Example 2

A rectifying element of Working Example 2 was obtained under the same conditions as in Working Example 1, except that the first electrode layer 21a, organic semiconductor material layer 30, and second electrode layer 21b were formed so as to have a thickness of 100 nm, 40 nm, and 100 nm, respectively.

### Working Example 3

A rectifying element of Working Example 3 was obtained by using a compound of the structural formula (I-13) shown below as a quinomethane-based compound and forming each deposited layer under the same conditions as in Working Example 1 to a thickness such that the first electrode layer 21a, organic semiconductor material layer 30, and second electrode layer 21b had a thickness of 100 nm, 80 nm, and 100 nm, respectively.

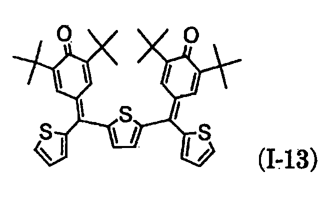

### Working Example 4

A rectifying element of Working Example 4 was obtained by using a compound of the structural formula (I-4) shown below as a quinomethane-based compound and forming each deposited layer under the same conditions as in Working Example 1 to a thickness such that the first electrode layer 21a, organic semiconductor material layer 30, and second electrode layer 21b had a thickness of 100 nm, 80 nm, and 100 nm, respectively.

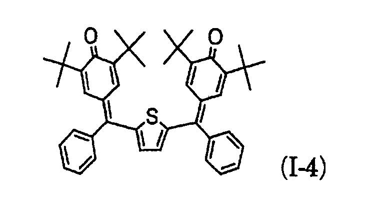

### Working Example 5

A rectifying element of Working Example 5 was obtained by using a compound of the structural formula (I-5) shown below as a quinomethane-based compound and forming each deposited layer under the same conditions as in Working Example 1 to a thickness such that the first electrode layer 21a, organic semiconductor material layer 30, and second electrode layer 21b had a thickness of 100 nm, 80 nm, and 100 nm, respectively.

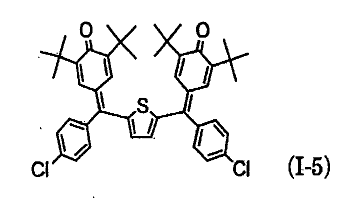

### Working Example 6

A rectifying element of Working Example 6 was obtained by using a compound of the structural formula (I-15) shown below as a quinomethane-based compound and forming each deposited layer under the same conditions as in Working Example 1 to a thickness such that the first electrode layer 21a, organic semiconductor material layer 30, and second electrode layer 21b had a thickness of 100 nm, 80 nm, and 100 nm, respectively.

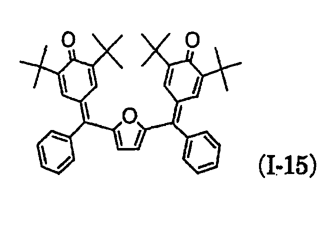

### Working Example 7

A rectifying element of Working Example 7 was obtained by using a compound of the structural formula (I-20) shown below as a quinomethane-based compound and forming each deposited layer under the same conditions as in Working Example 1 to a thickness such that the first electrode layer 21a, organic semiconductor material layer 30, and second electrode layer 21b had a thickness of 100 nm, 80 nm, and 100 nm, respectively.

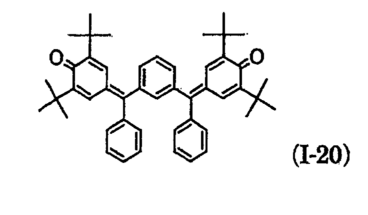

### Working Example 8

A rectifying element of Working Example 8 was obtained by forming films under the same conditions as in Working Example 1, except that the monoquinomethane-based compound of the structural formula (II-1) shown below was used for the organic semiconductor material layer 30.

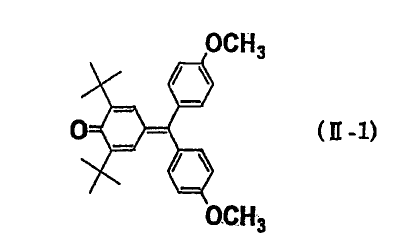

### Working Example 9

A rectifying element of Working Example 9 was obtained by forming films under the same conditions as in Working Example 1, except that the monoquinomethane-based compound of the structural formula (II-2) shown below was used for the organic semiconductor material layer 30.

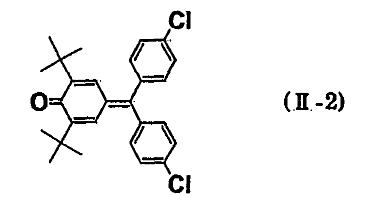

### Working Example 10

A rectifying element of Working Example 10 was obtained by forming films under the same conditions as in Working Example 1, except that the monoquinomethane-based compound of the structural formula (III-I) shown below was used for the organic semiconductor material layer 30.

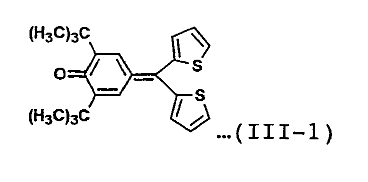

### Working Example 11

A rectifying element of Working Example 11 was obtained by forming films under the same conditions as in Working Example 1, except that the monoquinomethane-based compound of the structural formula (III-2) shown below was used for the organic semiconductor material layer 30.

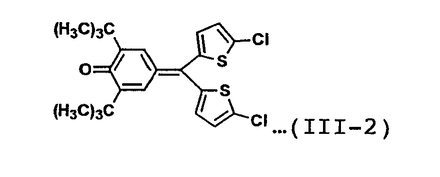

### Working Example 12

A rectifying element of Working Example 12 was obtained by forming films under the same conditions as in Working Example 1, except that the monoquinomethane-based compound of the structural formula (III-9) shown below was used for the organic semiconductor material layer 30.

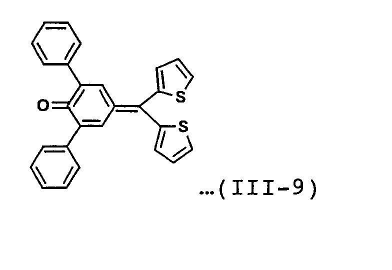

### Test Example 1

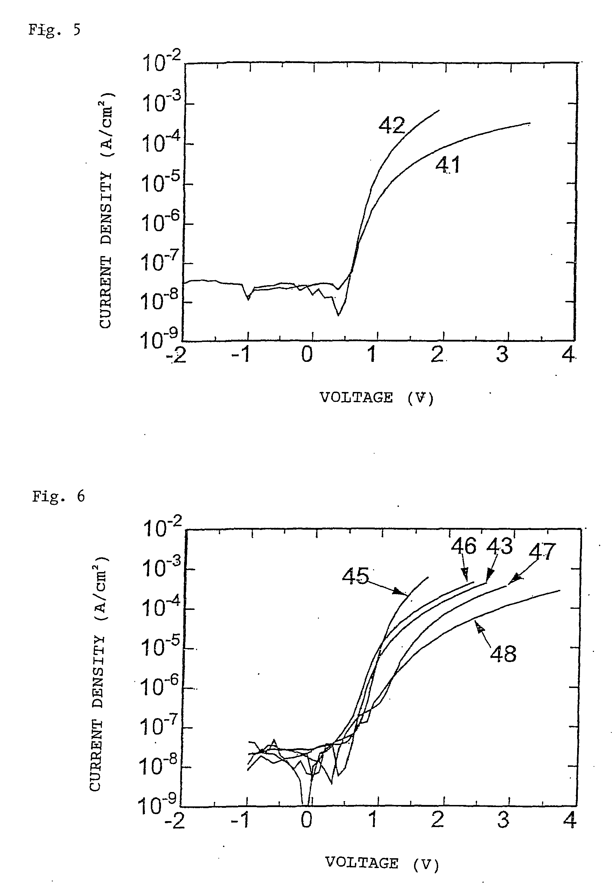

A current-voltage characteristic was measured in room temperature environment for the rectifying elements of above-described Working Examples 1 to 9. FIGS. 5 to 7 show the current-voltage characteristics relating to the rectifying elements of Working Examples 1 to 9, respectively. In the figures, the reference numerals 41-50 represent the current-voltage characteristics of Working Examples 1 to 9, respectively. The voltage plotted against the abscissa represents the difference in potential between the second electrode 21b and first electrode 21a.

The results shown in FIGS. 5 to 7 show that in most working examples the first electrode layer and second electrode are of the same material and though such a configuration is usually considered as unsuitable for obtaining an asymmetrical characteristic, a rectification characteristic was obtained in all the elements of Working Examples 1 to 9.

Thus, FIGS. 5 to 7 demonstrate that in all the elements of Working Examples 1 to 9, when a positive voltage was applied to the second electrode layer 21b, the electric current rose steeply with a threshold voltage of about 0.5 V as a boundary and a current of 10⁻⁵-10⁻⁶ A/cm² was reached at 1 V. By contrast, when a negative voltage was applied to the second electrode layer 21b, the current in all the cases was about 10⁻⁸ A/cm², that is, at a measurement noise level, even at -1 V and practically no current was flowing. Therefore, a rectification characteristic was obtained.

### INDUSTRIAL APPLICABILITY

With the present invention, a rectifying element of a simple structure and no difference in contact electric potential between the electrodes can be obtained. Therefore, the rectifying element of the present invention can be advantageously used in organic light-emitting displays or organic solar cells.

## Claims

1. A rectifying element in which an organic semiconductor material is disposed at least between two electrodes, wherein a first electrode layer, an organic semiconductor material layer, and a second electrode layer are successively formed as thin films on a substrate and the organic semiconductor material constituting said organic semiconductor material layer is a quinomethane-based compound represented by the general formula (I) below: 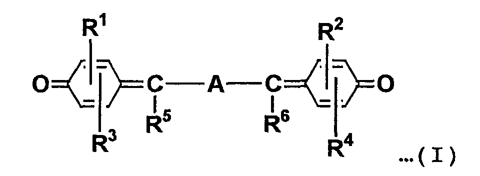 (in Formula (1), each of R¹-R⁴ represents a group selected from a hydrogen atom, an optionally substituted C₁₋₆ alkyl group, and an optionally substituted aryl group, where R¹-R⁴ may be same or different; each of R⁵ and R⁶ represents an optionally substituted aryl group or an optionally substituted hetero ring, where R⁵, R⁶ may be same or different; and a represents a group selected from groups (1)-(10) shown below).

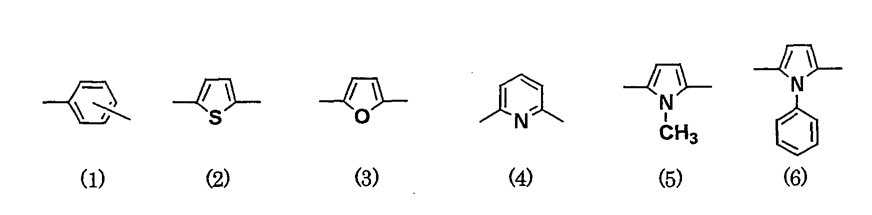

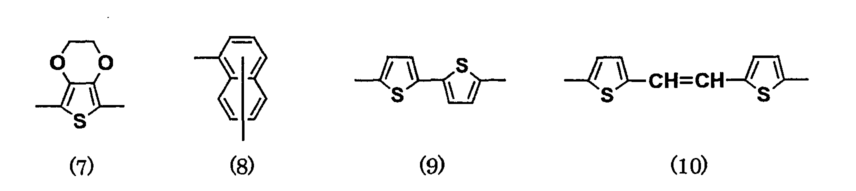

2. A rectifying element in which an organic semiconductor material is disposed at least between two electrodes, wherein a first electrode layer, an organic semiconductor material layer, and a second electrode layer are successively formed as thin films on a substrate and the organic semiconductor material constituting said organic semiconductor material layer is a monoquinomethane-based compound represented by the general formula (II) below: 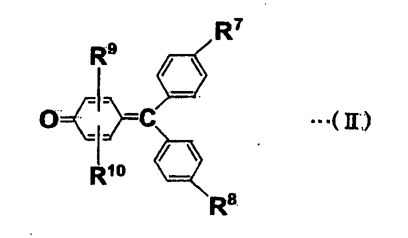 (in Formula (II), each of R⁷ and R⁸ represents a hydrogen atom, a halogen atom, a C₁₋₆ alkyl group, or an alkoxyl group, where R⁷ and R⁸ may be same or different; each of R⁹ and R¹⁰ represents a hydrogen atom, an optionally substituted C₁₋₆ alkyl group, or an optionally substituted aryl group, where R⁹ and R¹⁰ may be same or different).

3. A rectifying element in which an organic semiconductor material is disposed at least between two electrodes, wherein a first electrode layer, an organic semiconductor material layer, and a second electrode layer are successively formed as thin films on a substrate and the organic semiconductor material constituting said organic semiconductor material layer is a monoquinomethane-based compound represented by the general formula (III) below: 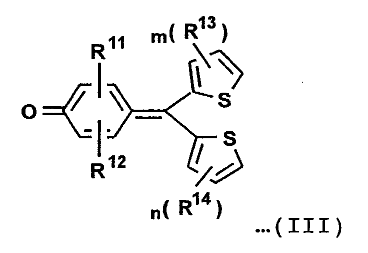 (in Formula (III), each of R¹¹-R¹⁴ represents a group selected from a hydrogen atom, a halogen atom, an optionally substituted C₁₋₆ alkyl group, and an optionally substituted aryl group, where R¹¹-R¹⁴ may be same or different; and m and n are integers, each from 0 to 3).

4. A rectifying element according to any one of claims 1 to 3, wherein said first electrode and said second electrode comprise the same material.

5. A rectifying element according to any one of claims 1 to 4, wherein said first electrode and said second electrode comprise aluminum.
